(19) Europäisches Patentamt
European Patent Office
Office européen des brevets

(11) Veröffentlichungsnummer: **0 002 525**
**B1**

(12) # EUROPÄISCHE PATENTSCHRIFT

(45) Veröffentlichungstag der Patentschrift: **29.04.81**

(51) Int. Cl.³: **C 07 F 9/53, C 07 C 31/04**

(21) Anmeldenummer: **78101677.9**

(22) Anmeldetag: **14.12.78**

(54) **Verfahren zur Aufbereitung von methanolisch-wässrigen Rückständen bei Synthesen mit Hilfe von Triphenylphosphoniumsalzen.**

(30) Priorität: **17.12.77 DE 2756321**

(43) Veröffentlichungstag der Anmeldung:
**27.06.79 Patentblatt 79/13**

(45) Bekanntmachung des Hinweises auf die Patenterteilung:
**29.04.81 Patentblatt 81/17**

(84) Benannte Vertragsstaaten:
**BE CH DE FR GB NL**

(56) Entgegenhaltungen:
**DE - A - 2 526 129**
**DE - C - 1 518 590**

(73) Patentinhaber: **BASF Aktiengesellschaft**
**Carl-Bosch-Strasse 38**
**D-6700 Ludwigshafen (DE)**

(72) Erfinder: **Grafen, Paul, Dr.**
**Suedtiroler Ring 18-20**
**D-6719 Weisenheim (DE)**
Erfinder: **Scholz, Hans-Ulrich, Dr.**
**Suedtiroler Ring 8**
**D-6719 Weisenheim (DE)**
Erfinder: **Schulz, Bernard, Dr.**
**Kurpfalzring 28**
**D-6830 Schwetzingen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 002 525 B1

Courier Press, Leamington Spa, England.

# Verfahren zur Aufbereitung von methanolisch-wäßrigen Rückständen bei Synthesen mit Hilfe von Triphenylphosphoniumsalzen

Die vorliegende Erfindung betrifft ein Verfahren zur Aufbereitung von hauptsächlich aus Methanol, Triphenylphosphinoxid, Salzen und organischen Nebenprodukten bestehen den Rückständen, die bei der Olefin-Synthese mit Hilfe von Triphenylphosphoniumsalzen nach Abtrennung der Olefine anfallen.

Bei diesen Synthesen, die — wie die Wittig-Synthese — großtechnisch vor allem zur Herstellung von Polyenen aus der Carotinoidreihe oder von deren Vorstufen dient, erhält man primär Reaktionsgemische, die sich im wesentlichen aus einem organischen Lösungsmittel und den Produkten der rechten Seite der Reaktionsgleichung zusammensetzen:

$$[R^1—P(Ph)_3]^{\oplus}\ X^{\ominus} + R^2—CO—R^3 + \text{Base} \rightarrow R^1 = C\begin{matrix} R^2 \\ R^3 \end{matrix}$$

$$+ PO(Ph)_3 + \text{Salz aus Base und HX} + \text{Nebenprodukte.}$$

In dieser Gleichung bedeuten Ph die Phenylgruppe, $X^{\ominus}$ das Säureäquivalent des Phosphoniumkations, $R^1$ den Ylidrest, meistens eine aliphatische oder cycloaliphatisch-aliphatische Polyenylgruppe mit 5 bis 40, vorzugsweise 5 bis 20 Rr/Fe C-Atomen, $R^2$ einen im allgemeinen ungesättigten organischen Rest und $R^3$ Wasserstoff oder ebenfalls einen organischen Rest. Je nach Art der Base kann das Reaktionsgemisch auch noch Wasser enthalten, sei es, daß man eine wäßrige Base verwendet oder daß Wasser bei der Salzbildung, z.B. bei Verwendung eines Alkalihydroxids, entsteht. Im Falle der technisch ausgeübten Vitamin-A-Synthes haben die genannten organischen Reste folgende aus dem Formelbild zu entnehmende Bedeutung:

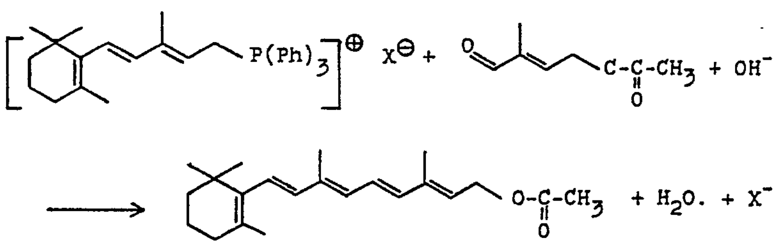

$$+ O = P(Ph)_3 + \text{Nebenprodukte.}$$

Auch bei anderen technischen Synthesen von Carotinoiden, wie der oxidativen Dimerisierung von Polyenyltriphenylphosphoniumsalzen gemäß DE - AS 25 05 869 fallen neben den gewünschten Olefinen Triphenylphosphinoxid und Salze gemäß der folgenden Formel an:

$$2[R^1—P(Ph)_3]^{\oplus}\ X^{\ominus} + H_2O_2 + \text{Base} \rightarrow$$

$$R^1 = R^1 + 2\ O = P(Ph)_3 + \text{Salze aus Base und HX}$$

$$+ \text{Nebenprodukte.}$$

Zur Gewinnung des Wertproduktes, des Olefins, versetzt man dieses Primärgemisch mit reinem oder wäßrigem Methanol und extrahiert im allgemeinen aus diesem Gemisch das Olefin mit einem hydrophoben Lösungsmittel, wie z.B. Heptan. Statt durch Extraktion können schwerlösliche Carotinoide auch durch Filtration abgetrennt werden.

Nach dieser Extraktion oder Filtration verbleiben methanolische oder wäßrig-methanolische Lösungen, die hauptsächlich Triphenylphosphinoxid, das Salz aus der Säure HX und der verwendeten Base, gegebenenfalls ein weiteres organisches Lösungsmittel sowie diverse organische Nebenprodukte enthalten. Diese Lösungen lassen sich nur mit hohem apparativen Aufwand aufarbeiten, da beim Eindampfen dieser Mischungen zuerst das Methanol, das eigentliche Lösungsmittel für das Triphenylphosphinoxid, abdestilliert. Dadurch fällt das Triphenylphosphinoxid in der wäßrigen Salzphase ölig oder feinkristallin aus. Beim weiteren Eindampfen wird durch das teilweise Abdestillieren von Wasser die Konzentration an anorganischen Salzen so hoch, daß diese beginnen auszufallen. Normale Verdampfer verstopfen dadurch sehr schnell. Der Destillationsrückstand einer solchen schwierig zu handhabenden Destillation besteht aus einer Mischung von anorganischen Salzen, kristallinem Triphenylphosphinoxid mit eingeschlossenen Nebenprodukten. Um aus diesem Rückstand das Triphenylphosphinoxid in eine für die Rückgewinnung zum Triphenylphosphin gemäß DE-PS 1 247 310 und DE-PS 1 192 205 oder gemäß U.S. Patent 3 847 399 geeignete Form zu bringen, sind aufwendige Reinigungsoperationen notwendig. Eine Verbrennung dieses Rückstandes ist teuer und aufwendig wegen des hohen Anteils an anorganischen Salzen.

Der Erfindung lag daher die Aufgabe zugrunde, die bei der Wittig-Synthese bzw. die bei der oxidativen Dimerisierung von Phosphoniumsalzen nach der Extraktion oder Abtrennung der Olefine anfallenden methanolischen Rückstände wirtschaftlicher als bisher aufzubereiten.

Es wurde nun ein Verfahren zur Aufbereitung von Rückständen, wie sie bei Wittig'schen Olefinsynthesen in Methanol als Lösungsmittel bzw. bei der oxidativen Dimerisierung von Phosphoniumsalzen nach der Extraktion oder Abtrennung der Olefine anfallen und die hauptsächlich aus Methanol, Triphenylphosphinoxid, Salzen, organischen Nebenprodukten und ggf. Wasser bestehen, zwecks Reinigung des Abwassers von Triphenylphosphinoxid und anderen organischen Verunreinigungen und Rückgewinnung des Triphenylphosphinoxids gefunden, das dadurch gekennzeichnet ist, daß man

a) aus diesen Rückstanden die flüchtigen Bestandteile, wie hauptsächlich das Methanol, mit Wasserdampf bei Temperaturen von 80 bis 200°C austreibt,
b) daß man bei der Wasserdampfbehandlung so arbeitet, daß mindestens so viel Wasserdampf im Rückstand zu Wasser kondensieren kann, daß die im Rückstand enthaltenen Salze in Lösung bleiben, die Wasserphase jedoch möglichst gering bleibt,
c) daß man das sich bildende wäßrig-organische Gemisch in ein Beruhigungsgefäß überführt, wo man es bei 80 bis 100°C hält, und
d) daß man die sich hierbei ausbildenden Phasen aus einer salzhaltigen wäßrigen Lösung und einer hauptsächlich Triphenylphosphinoxid enthaltenden organischen Lösung voneinander trennt und in an sich bekannter Weise weiterverarbeitet.

Das unerwartet gute Gelingen dieses Verfahrens beruht auf der überraschenden Beobachtung, daß das Triphenylphosphinoxid unter den angegebenen Bedingungen mit Wasser offensichtlich in sein Hydrat übergeht, welches unter 100°C schmilzt und ein ausgezeichnetes Lösungsvermögen für organische Substanzen hat. Demgemäß bilden sich zwei flüssige Phasen, nämlich eine wäßrige, die den Großteil der salzartigen Bestandteile, Spuren Methanol und sehr geringe Mengen organischer Substanzen enthält, sowie eine schwerere organische Phase, die im wesentlichen aus dem Triphenylphosphinoxidhydrat und organischen Nebenprodukten besteht, die sich bei der Olefinierungsreaktion gebildet haben.

Das Verfahren ist nach den bisherigen Beobachtungen auf alle Synthesen, die Triphenylphosphoniumsalze als Ausgangsverbindung benutzen, zur Herstellung beliebiger Olefine anwendbar, so daß weder die Natur der oben angegebenen Reste $R^1$ bis $R^3$ noch des Säurerestes $X^{\ominus}$ noch der Base einen Einfluß auf seine Anwendbarkeit hat. Detaillierte Ausführungen über die Reaktionspartner derartiger Synthesen sind daher entbehrlich. Es sei lediglich hervorgehoben, daß es seine größte technische Bedeutung im Falle von Synthesen von Verbindungen der Carotinoidreihe, wie dem Vitamin A und dem $\beta$-Carotin hat.

Demgemäß sind die Ylidreste $R^1$ vorzugsweise aliphatische oder cycloaliphatisch-aliphatische Polyenylgruppen mit 5 bis 20 C-Atomen, wie vor allem die 3-Methyl-4-dimethoxy-2-buten-1-yl-, die $\beta$-Jonylidenäthyl- und die Axerophthylgruppe:

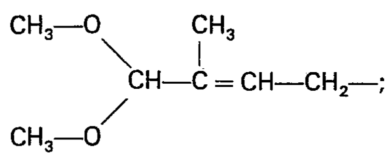

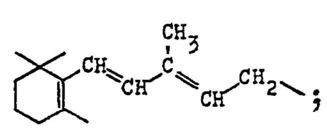

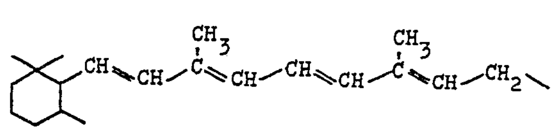

Die zur Phosphoniumsalzbildung üblicherweise verwendeten Säuren sind Schwefelsäure, Salpetersäure, HCl, HBr, Essigsäure, Ameisensäure, Propionsäure, Toluolsulfonsäuren, Oxalsäure, Trichloressigsäure und die sauren Salze mehrbasischer Säuren, wie Hydrogensulfate und andere.

Im Hinblick auf das erfindungsgemäße Verfahren kommen indes auch alle Säuren in Betracht, die in der Lage sind, Triphenylphosphoniumsalze zu bilden.

Säuren mit größeren organischen Resten sind jedoch weniger geeignet, weil das Abwasser möglichst frei von organischen Bestandteilen sein soll.

Als Carbonylverbindungen gehen hauptsächlich Aldehyde ($R^3$ = H) in die Synthese ein. Die organischen Reste $R^2$ können zur Klasse der Reste $R^1$ gehören oder auch Reste mit geringerer Anzahl von C-Atomen sein. Häufig tragen diese Reste funktionelle Gruppen wie freie oder mit niederen Carbonsäuren veresterte Hydroxylgruppen. Als technisch besonders wichtige Carbonylverbindungen seien genannt: $\gamma$-Acetoxytiglinaldehyd, $\beta$-Formylcrotonsäureäthylester, 2,7-Dimethyl-octa-2,4,6-trien-1,8-dial, Retinal, $\beta$-Apo-12'-carotinal, $\beta$-Apo-8'-carotinal und deren Homologe.

Die für die Olefinbildung gebräuchlichsten Basen sind wäßrige oder wasserfreie Alkali- oder Erdalkalihydroxide sowie wäßriger oder wasserfreier Ammoniak, die Alkali- und Erd-alkalicarbonate, vorzugsweise Natrium- und Kaliumcarbonat, sowie basisches Magnesiumcarbonat und Calciumcarbonat. Mit besonderem Vorteil verwendet man KOH oder NaOH, LiOH, $Mg(OH)_2$, $Ca(OH)_2$ oder $Ba(OH)_2$. Organische Amine wie Dimethylamin oder Träthylamin sind zwar ebenfalls geeignet, bringen aber nur ausnahmsweise Vorteile im Vergleich zu den billigen Basen. Im Hinblick auf die Rückstandsaufbereitung sind die organischen Amine weniger erwünscht, da das Abwasser wegen der organischen Anteile dann noch einer gesonderten Behandlung bedarf und nicht ohne weiteres verworfen werden kann.

Die Bildung der Phosphoniumsalze sowie die anschließende Ylid-Reaktion werden in aller Regel in einem inerten organischen Lösungsmittel ausgeführt. Falls es die Löslichkeit der Komponenten gestattet, bevorzugt man ein billiges, leicht rückgewinnbares Lösungsmittel wie Methanol, zumal sich dieses für den anschließenden Schritt der Triphenylphosphinoxidextraktion am besten bewährt hat. Es kommen aber auch andere Lösungsmittel wie Äthanol, Propanole, Butanole, Glykole und Glykoläther sowie Tetrahydrofuran, Dioxan und Methylenchlorid in Betracht. die zum Teil, wie beispielsweise das Methylenchlorid, vor der Extraktion destillativ entfernt werden. Wenn sie bei der Extraktion nicht stören, wie dies z.B. bei Äthanol oder Isopropanol der Fall ist, kann man sie aber auch eventuell beim erfindungsgemäßen Schritt der Wasserdampfbehandlung zusammen mit dem Methanol entfernen.

Für die Extraktion haben sich methanolische Lösungen am besten bewährt, da aus ihnen das gewünschte Wertprodukt, nämlich das Olefin, besonders einfach zu isolieren ist.

Als Extraktionsmittel verwendet man vorzugsweise einen unpolaren Kohlenwasserstoff niederen Siedepunkts wie Heptan, Hexan, Pentan, Octan und deren Isomerengemische. Aber auch Cyclohexan und Methylcyclohexan oder aromatische Kohlenwasserstoffe, wie Toluol, Xylol und Benzol können eingesetzt werden. Die verbleibenden Rückstände setzen sich bei den üblichen Synthesen in der Carotinoidreihe, z.B. zur Herstellung von Vitamin-A-Acetat, etwa wie folgt zusammen (jeweils Gew.%).

| | | |
|---|---|---|
| Methanol | 0 | bis 80 Gew.% |
| Triphenylphosphinoxid | 5 | bis 80 Gew.% |
| Salze (z.B. $Na_2SO_4$) | 1 | bis 40 Gew.% |
| organische Nebenprodukte | 0,01 | bis 20 Gew.% |
| Wasser | 0 | bis 90 Gew.% |
| Lösungsmittel außer Methanol | 0 | bis 80 Gew.% |

Diese Werte sind nicht kritisch und variieren von Fall zu Fall. Bei speziellen Wittig-Synthesen außerhalb der Carotinoid-Chemie mag die Zusammensetzung der Rückstandslösungen erheblich von den genannten Werten abweichen, jedoch hat dies keinen nennenswerten Einfluß auf das erfindungsgemäße Verfahren.

Zur praktischen Durchführung des erfindungsgemäßen Verfahrens werden das Methanol und gegebenenfalls auch das weitere Lösungsmittel, zunächst bei 80 bis 200°C (bei Temperaturen oberhalb 100°C, also unter entsprechendem Überdruck) mit Wasserdampf ausgetrieben, wobei man es so einrichtet, daß etwa das Volumen des Methanols durch Wasser, also kondensierten Wasserdampf, ersetzt wird. Damit wird erreicht, daß einerseits alle unter diesen Bedingungen abdestillierbaren organischen Lösungsmittel vollständig abdestilliert werden und andererseits die im Rückstand enthaltenen Salze in Lösung bleiben. Selbstverständlich wird man bestrebt sein, die Wasserphase möglichst gering zu halten. Die für den Verfahrenserfolg optimale Wassermenge läßt sich anhand einiger Vorversuche unschwer ermitteln; die Maßgabe, daß Methanol- und Wasservolumen ungefähr gleich sein sollen, stellt somit lediglich einen Richtwert dar.

Nach der Wasserdampfbehandlung, bei der alle Komponenten des Rückstandes kräftig durchmischt werden und während der sich das für das Verfahren wichtige Phosphinoxid-Hydrat bildet, über dessen genaue chemische Natur nichts näheres bekannt ist, überführt man das Gemisch in ein Beruhigungsgefäß, wobei sich bei 80 bis 100°C zwei flüssige Phasen ausbilden. Es war überraschend,

daß sich zwei flüssige Phasen ausbildeten, da wegen des hohen Schmelzpunktes des Triphenylphosphinoxids von 156°C ein Kristallisieren erwartet wurde, was eine Abtrennung sehr erschwert oder unmöglich gemacht hätte. Diese beiden flüssigen Phasen werden wie üblich getrennt und gesondert weiterverarbeitet. Die wäßrige salzhaltige Phase wird in aller Regel verworfen, es sei denn, sie enthielte besonders wertvolle Salze, die deren Rückgewinnung lohnend machten. Der organische Anteil der wäßrigen Phase ist mit 0 bis 1% überraschend gering und belastet somit kaum noch die für Industrieabwässer erforderliche chemisch-biologische Abwasserreinigung.

Die untere organische Phase besteht zu 70 bis 90 Gew.% aus Triphenylphosphinoxid, 4 bis 6 Gew.% Wasser und zu 26 bis 4 Gew.% aus organischen Verunreinigungen aus der Olefin-Synthese. Es war überraschend, daß die Triphenylphosphinoxid-haltige Phase trotz ihres hohen Wassergehaltes (50 Mol%) ein so starkes Lösevermögen für organische Stoffe besitzt. Ob man die organische Phase verbrennt oder das Triphenylphosphinoxid daraus destillativ oder durch Umkristallisieren zurückgewinnt, ist eine Frage der Wirtschaftlichkeit. In aller Regel wird sich jedoch die Rückgewinnung lohnen, da die Phosphorverbindung wieder in das wertvolle Triphenylphosphin überführt werden kann. Lediglich der danach verbleibende Rückstand ist meistens wertlos und wird deshalb verbrannt oder anderweitig vernichtet.

Man kann das erfindungsgemäße Verfahren nach den üblichen Methoden diskontinuierlich oder kontinuierlich ausführen. Da technische Besonderheiten hierbei nicht zu beachten sind, erübrigen sich nähere Ausführungen hierüber. Besonders zweckmäßig erfolgt die erfindungsgemäße Wasserdampfbehandlung in einer Kolonne, an deren Kopf das Gemisch aus Methanol, Wasser, Triphenylphosphinoxid, Salzen und organischen Nebenprodukten zuläuft und in deren Sumpf der Dampf eingeleitet wird.

Mit Hilfe des erfindungsgemäßen Verfahrens ist es möglich, die Abwässser von Synthesen, bei denen Triphenylphosphinoxid anfällt, auf einfache Weise von Triphenylphosphinoxid und anderen organischen Verunreinigungen zu befreien, wodurch einerseits die Abwasserklärung wesentlich erleichtert wird und andererseits ein einfacher Weg zur Rückgewinnung des Triphenylphosphinoxids aufgezeigt wird. Das durch Destillation oder Umkristallisieren leicht zu reinigende Triphenylphosphinoxid kann dann z.B. zur Herstellung des für Wittig-Synthesen essentielle Triphenylphosphins, zur Herstellung des zur Synthese von Schädlingsbekämpfungsmitteln notwendige Triphenylphosphindichlorids, als "Flame-retardant" oder als Extraktionsmittel für organische Stoffe verwendet werden. Der Einsatz von Triphenylphosphinoxid als Extraktionsmittel bringt große Vorteile mit sich, da es einerseits schwerentflammbar ist und andererseits wegen seines hohen Siedepunktes (380°C) eine leichte Abtrennung tiefer siedender Substanzen erlaubt.

## Beispiel 1

Bei der Umsetzung von 562 g (1 Mol) β-Jonylidenäthyltriphenylphosphoniumhydrogensulfat in 1 l Wasser mit 180 g (1,27 Mol) γ-Acetoxytiglinaldehyd und einer wäßrigen Lösung von 207 g Kaliumcarbonat und Aufarbeiten des Reaktionsansatzes mit Methanol und Hepten erhielt man neben dem Wertprodukt Vitamin-A-acetat einen Rückstand, der im wesentlichen folgende Zusammensetzung hatte:

1 850 g Wasser

2 240 g Methanol

278 g Triphenylphosphinoxid

175 g Kaliumsulfat

60—70 g Kaliumcarbonat

sowie organische Verunreinigungen.

Durch Einblasen von 2,5 bis 3 kg Wasserdampf in diesen Rückstand wurde das gesamte Methanol in Form einer etwa 50 gewichtsprozentigen Mischung mit Wasser abgetrieben. Die Zusammensetzung des erhaltenen Destillats ist apparateabhängig.

Der nach der Wasserdampfbehandlung anfallende Rückstand trennte sich in eine obere wäßrige Phase, in der die anorganischen Salze gelöst und in eine oberhalb 80°C flüssige organische Phase, die Triphenylphosphinoxid und die in dem ursprünglichen methanolisch-wäßrigen Extraktionsrückstand vorhandenen nichtflüchtigen organischen Nebenprodukte enthielt.

Die wäßrige Phase bestand aus etwa 175 g Kaliumsulfat, 60 bis 70 g Kaliumcarbonat und nur Spuren organischen Materials in 2,6 kg Wasser und konnte somit problemlos zur Abwasserbehandlung gegeben werden.

Die untere organische Phase bestand aus 300 g eines Öles, das aufgrund der Phosphoranalyse etwa folgende Zusammensetzung hatte: ca. 278 g Triphenylphosphinoxid und 22 g größtenteils organische Nebenprodukte. Das Triphenylphosphinoxid wird bei Temperaturen unterhalb von 80°C kristallin und kann nach Reinigung zur Rückgewinnung von Triphenylphosphin nach bekannten Verfahren eingesetzt werden.

Beispiel 2

1 582 Gewichtsteile eines Olefin-Synthese-Rückstandes etwa folgender Zusammensetzung:

763 Gewichtsteile Wasser

571 Gewichtsteile Methanol

101 Gewichtsteile Triphenylphosphinoxid

123 Gewichtsteile anorganische Salze

120 Gewichtsteile organische Nebenprodukte und

3 Gewichtsteile Wasserstoffperoxid

wurden erfindungsgemäß aufgearbeitet.

Dazu wurden die 1 582 Gewichtsteile in 1 Stunde auf eine entsprechend dimensionierte Füllkörperkolonne gegeben, die mit 871 Gewichtsteilen Dampf pro Stunde direkt beheizt wurde. Man erhielt in einer Stunde als Destillat eine Mischung von 568 Teilen Methanol und 571 Teilen Wasser.

Als ca. 99°C heißen Sumpfablauf erhält man in 1 Stunde 1 313 Gewichtsteile einer zweiphasigen Mischung etwa folgender Zusammensetzung:

1 063 Gewichtsteile Wasser

101 Gewichtsteile Triphenylphosphinoxid

123 Gewichtsteile Salz

20,5 Gewichtsteile organische Verunreinigung und

3 Gewichtsteile Methanol (~0,1%).

Dieser heiße Sumpfablauf wurde kontinuierlich in ein beheiztes Trenngefäß (Phasenscheider) geführt. Man erhielt in 1 Stunde 1 179 Teile einer als Oberphase ablaufenden Lösung aus 1 054 Gewichtsteilen Wasser, 122,4 Gewichtsteilen anorganischen Salzen, 1 Teil Triphenylphosphinoxid, 0,5 Gewichtsteilen organischen Verunreinigungen und 1 Gewichtsteil Methanol. Als Unterphase laufen in 1 Stunde 129,6 Gewichtsteile einer oberhalb 80°C flüssigen Mischung aus 100 Gewichtsteilen Triphenylphosphinoxid, 7 Gewichtsteilen Wasser, 0,6 Gewichtsteilen anorganischen Salzen, 20 Gewichtsteilen organischen Verunreinigungen und 2 Gewichtsteilen Methanol ab. Diese Phase kann der Aufarbeitung zu Triphenylphosphin zugeführt werden.

**Patentanspruch**

Verfahren zur Aufbereitung von Rückständen, wie sie bei Wittig'schen Olefinsynthesen in Methanol als Lösungsmittel bzw. bei der oxidativen Dimerisierung von Phosphoniumsalzen nach der Extraktion oder Abtrennung der Olefine anfallen und die hauptsächlich aus Methanol, Triphenylphosphinoxid, Salzen, organischen Nebenprodukten und ggf. Wasser bestehen, zwecks Reinigung des Abwassers von Triphenylphosphinoxid und anderen organischen Verunreinigungen und Rückgewinnung des Triphenylphosphinoxids, dadurch gekennzeichnet, daß man

a) aus diesen Rückständen die flüchtigen Bestandteile, wie hauptsächlich das Methanol, mit Wasserdampf bei Temperaturen von 80 bis 200°C austreibt,
b) daß man bei der Wasserdampfbehandlung so arbeitet, daß mindestens so viel Wasserdampf im Rückstand zu Wasser kondensieren kann, daß die im Rückstand enthaltenen Salze in Lösung bleiben, die Wasserphase jedoch möglichst gering bleibt,
c) daß man das sich bildende wäßrig-organische Gemisch in ein Beruhigungsgefäß überführt, wo man es bei 80 bis 100°C hält, und
d) daß man die sich hierbei ausbildenden Phasen aus einer salzhaltigen wäßrigen Lösung und einer hauptsächlich Triphenylphosphinoxid enthaltenden organischen Lösung voneinander trennt und in an sich bekannter Weise weiterverarbeitet.

**Revendication**

Procédé de traitement de mélanges résiduels, provenant de synthèses d'oléfines d'après Wittig dans le méthanol comme solvant, ou de la dimérisation oxydante de sels de phosphonium après l'extraction ou la séparation des oléfines, et formés principalement de méthanol, d'oxyde de triphényl-phosphine, de sels, de produits organiques secondaires et éventuellement d'eau, traitement destiné à extraire des eaux résiduaires l'oxyde de triphényl-phosphine et d'autres impuretés organiques et à récupérer l'oxyde de triphényl-phosphine, caractérisé en ce que:

a) on chasse les ingrédients volatils du mélange résiduel, en particulier le méthanol, entre 80 et 200°C par entraînement à la vapeur d'eau;
b) on réalise le traitement à la vapeur d'eau dans des conditions opératoires telles qu'une fraction suffisante pour maintenir les sels contenus dans le mélange résiduel en solution de cette vapeur d'eau se condense en eau dans le mélange, la phase aqueuse étant cependant maintenue aussi réduite que possible;
c) on transfère le mélange aqueux-organique formé dans un séparateur de phases, où il est maintenu au repos entre 80 et 100°C, et
d) on sépare la phase aqueuse contenant les sels de la phase organique contenant principalement l'oxyde de triphényl-phosphine et on soumet chaque phase séparément aux opérations subséquentes de manière connue en soi.

**Claim**

A process for the treatment of residues such as are obtained in the Wittig synthesis of olefins in methanol as solvent or in the oxidative dimerization of phosphonium salts, after extraction or isolation of the olefins, and which consist principally of methanol, triphenylphosphine oxide, salts and organic by-products, with or without water, for the purpose of freeing the aqueous effluent from triphenylphosphine oxide and other organic impurities, and recovering the triphenylphosphine oxide, characterized in that

a) these residues are steam-stripped at from 80 to 200°C to remove the volatile constituents, principally methanol,
b) the steam treatment is carried out in such a way that at least sufficient steam in the residue can condense to water that the salts contained in the residue stay in solution, but the amount of the aqueous phase is kept as small as possible,
c) the aqueous organic mixture formed is transferred to a settling vessel, where it is kept at 80 to 100°C, and
d) the phases which form in the vessel, namely a saline aqueous solution and an organic solution containing principally triphenylphosphine oxide, are separated from one another and processed further in a conventional manner.